# EUROPEAN PATENT SPECIFICATION

(11) **EP 4 274 521 B1**
(45) Date of publication and mention of the grant of the patent: **23.10.2024**
(21) Application number: 22701084.0
(22) Date of filing: 04.01.2022
(51) Int. Cl.: A61F 5/44, A61F 5/441, A61F 5/451

(54) **PORTABLE URINE COLLECTION SYSTEMS AND RELATED METHODS**
TRAGBARE URINSAMMELSYSTEME UND ENTSPRECHENDE METHODEN
SYSTÈMES PORTABLES DE COLLECTE D'URINE ET MÉTHODES ASSOCIÉES

(30) Priority: 06.01.2021 US 202163134287 P
(43) Date of publication of application: 15.11.2023
(73) Proprietor: Purewick Corporation, El Cajon, California 92020 (US)
(72) Inventor: YOUNG JOYNER, Melissa, Stone Mountain, Georgia 30087 (US); ABDELAL, Dana Ahmad, Marietta, Georgia 30067 (US); KULKARNI, Vinayaka, Bangalore Karnataka 56007 (IN); EARNSHAW, Audrey, Erie, Colorado 80516 (US); CHANCY, Patrick Hudson, Dunwoody, Georgia 30338 (US)
(74) Representative: Studio Torta S.p.A.
(86) International application number: PCT/US2022/011108
(87) International publication number: WO 2022/150290

(56) References cited:
- EP-A1- 1 872 752
- EP-A1- 2 389 908
- WO-A1-2004/019836
- WO-A1-2020/256865
- CN-U- 209 285 902
- US-A- 4 631 061
- US-A1- 2007 225 668
- US-A1- 2012 066 825
- US-A1- 2019 046 102
- US-A9- 2020 171 217
- US-B2- 7 131 964

## Description

### BACKGROUND

An individual may have limited or impaired mobility such that typical urination processes are challenging or impossible. For example, the individual may have surgery or a disability that impairs mobility. In another example, the individual may have restricted travel conditions such as those experience by pilots, drivers, and workers in hazardous areas. Additionally, fluid collection from the individual may be needed for monitoring purposes or clinical testing.

Bed pans and urinary catheters, such as a Foley catheter, may be used to address some of these circumstances. However, bed pans and urinary catheters have several problems associated therewith. For example, bed pans may be prone to discomfort, spills, and other hygiene issues. Urinary catheters be may be uncomfortable, painful, and may cause urinary tract infections. Conventional urine collection devices also may be limited to use when a patient is confined to a bed in a supine position.

Thus, users and manufacturers of fluid collection devices continue to seek new and improved devices, systems, and methods to collect urine. Examples of such devices, systems, and methods are disclosed in US2007225668A1, US4631061A, WO2004019836A1, EP1872752A1, US2012066825A1, EP2389908A1. Document EP 1 872 752 A1 discloses the features of the preamble of claim 1.

### SUMMARY

The invention is set out in the appended claims.

Embodiments disclosed herein are related to fluid collection devices and methods of using fluid collection devices. In an embodiment, a portable urine collection system includes a urine collection device, a first conduit, a urine collection container, a pump, a sensor, and a container support. The urine collection device is configured to be positioned at least proximate to a urethra of a user. The first conduit is in fluid communication with the urine collection device and the urine collection container has an interior region. The pump is in fluid communication with the urine collection container and is configured to pull a vacuum on the interior region of the urine collection container effective to draw urine from the urine collection device through the first conduit into the urine collection container. The sensor is secured to the container and configured to detect a property relating at least to a volume of the urine in the urine collection container. The cover is sized and dimensioned to hold at least the pump therein and obscure at least the pump from view outside the cover.

A method of assembling a portable urine collection system includes detachably securing a container support to a wheelchair. The method also includes mounting a urine collection container to the container support, the urine collection container including a sensor secured thereto and configured to detect a property relating at least to a volume of the urine in the urine collection container. The method also includes positioning a urine collection device proximate to a urethra of a user. The method also includes fluidly coupling the urine collection device to the urine collection container with a first conduit. The method also includes mounting a pump to the wheelchair with the pump in fluid communication with the urine collection container and configured to pull a vacuum on the interior region of the urine collection container effective to draw urine from the urine collection device through the first conduit into the urine collection container.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1** is a block diagram of a portable urine collection system, according to an embodiment.
**FIG. 2A** is a rear isometric view of a portable urine collection system secured to a wheelchair, according to an embodiment.
**FIG. 2B** is the portable urine collection system of **FIG. 2A** without the wheelchair.
**FIG. 2C** is a belt of the portable urine collection system, according to an embodiment.
**FIG. 3A** is a front isometric view of a portable urine collection system secured to a wheelchair, according to an embodiment.
**FIG. 3B** is a rear view of the portable urine collection system of **FIG. 3A** secured to a wheelchair.
**FIG. 3C** is a front isometric view of the portable urine collection system of **FIG. 3A** secured to a wheelchair with a seat of the wheelchair removed.
**FIG. 4A** is a top isometric view of the portable urine collection system of **FIG. 3A**, according to an embodiment.
**FIG. 4B** is a front isometric view of a urine collection container and a container cover of the portable urine collection system of **FIG. 3A****.**
**FIG. 4C** is a front isometric view of a urine collection container and a container cover of the portable urine collection system of **FIG. 3A** on a table, according to an embodiment.
**FIG. 5A** is a cross-sectional of the urine collection container and the container cover of the portable urine collection system of **FIG. 3A****,** according to an embodiment.
**FIG. 5B** is a block diagram of a controller in the urine collection system of **FIG. 3A****,** according to an embodiment.
**FIG. 6A** is a rear perspective view of a urine collection system secured to a wheelchair, according to an embodiment.
**FIG. 6B** is a cross-sectional view of the urine collection container of the urine collection system of **FIG. 6A****,** according to an embodiment.
**FIG. 7** is a flow diagram of a method for assembling a portable urine collection system, according to an embodiment.

### DETAILED DESCRIPTION

Embodiments disclosed herein are related to wheelchair securable urine collection systems and related methods. Many users of urine collection devices are over 65 years old with limited mobility, often relying on wheelchairs as a primary mode of transportation. Many users also spend a significant amount of their day in a seated or supine position. Users and caregivers, then, are benefited from a urine collection system that may be both discrete and mobile, allowing users to use the urine collection system to collect urine both at home and on the go.

In at least one, some, or all of the embodiments described herein, a urine collection system is compact and provides the technical effect resulting in securing or mounting the urine collection system to a wheelchair. In at least one, some, or all of the embodiments, the urine collection system also can by positioned or placed on a surface near the user in addition to being securable or mountable to a wheelchair, resulting in the technical effect of a urine collection system that is useable both with and without a wheelchair. At least one, some, or all of the embodiments of the urine collection systems described herein are mobile and discreet, resulting in the technical effect of allowing a user to participate in social activities without alerting others to the incontinence of the users. For example, the urine collection systems may include a cover configured to hold at least the pump of a urine collection system therein to obscure the pump from view outside the urine collection system. The urine collected in the urine collection systems described herein also may be stored in a urine collection container that is obscured from view outside the urine collection system and/or obscures the urine held in the urine collection container.

In many embodiments, the urine collection system also includes an alert system configured to communicate with an electronic device to alert a user or caregiver to empty the urine collection container when the urine level approaches a predetermined level, to change or recharge a battery, and/or adjust a vacuum or suction level of the pump in the urine collection system.

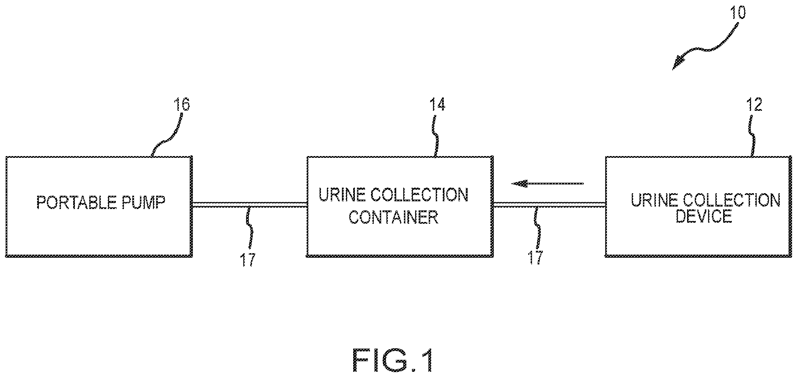

**FIG. 1** is a block diagram of a fluid collection system 10, according to an embodiment. The fluid collection system 10 may be included in any of the embodiments of fluid collection systems described herein. The system 10 includes a fluid (*e.g.,* urine) collection device 12 (*e.g.,* any of the fluid collection assemblies disclosed herein), a urine collection container 14, and a pump 16 (or pump). The fluid collection device 10, the urine collection container 14, and the pump 16 may be fluidly coupled to each other via one or more conduits 17. For example, fluid collection device 10 may be operably coupled to one or more of the urine collection container 14 or the pump 16 via the conduit 17. In some embodiments, the pump 16 may be secured directly to the urine collection container 14. Fluid (*e.g*., urine or other bodily fluids) collected in the fluid collection device 10 may be removed from the fluid collection device 10 via the conduit 17 secured to the fluid collection device 12. Suction force may be introduced into the chamber of the fluid collection device 12 via the inlet of the conduit 17 responsive to suction (*e.g*., vacuum) force applied at the outlet of the conduit 17.

The suction force may be applied to the outlet of the conduit 17 by the pump 16 either directly or indirectly. The suction force may be applied indirectly via the urine collection container 14. For example, the outlet of the conduit 17 may be disposed within or fluidly coupled to an interior region of the urine collection container 14 and an additional conduit 17 may extend from the urine collection container 14 to the pump 16. Accordingly, the pump 16 may apply suction to the fluid collection device 12 via the urine collection container 14. The suction force may be applied directly via the pump 16. For example, the outlet of the conduit 17 may be disposed within the pump 16. An additional conduit 17 may extend from the pump 16 to a point outside of the fluid collection device 12, such as to the urine collection container 14. In such examples, the pump 16 may be disposed between the fluid collection device 12 and the urine collection container 14.

The urine collection container 14 is sized and shaped to retain a fluid therein. The urine collection container 14 may include a bag (*e.g*., drainage bag), a bottle or cup (*e.g.,* collection jar), or any other enclosed container for storing bodily fluid(s) such as urine. In some examples, the conduit 17 may extend from the fluid collection device 12 and attach to the urine collection container 14 at a first point therein. An additional conduit 17 may attach to the urine collection container 14 at a second point thereon and may extend and attach to the pump 16. Accordingly, a vacuum (*e.g*., suction) may be drawn through fluid collection device 12 via the urine collection container 14. Fluid, such as urine, may be drained from the fluid collection device 12 using the pump 16.

The pump 16 may include one or more of a manual vacuum pump, and electric vacuum pump, a diaphragm pump, a centrifugal pump, a displacement pump, a magnetically driven pump, a peristaltic pump, or any pump configured to produce a vacuum. The pump 16 may provide a vacuum or suction to remove fluid from the fluid collection device 12. In some examples, the pump 16 may be powered by one or more of a power cord (*e.g*., connected to a power socket), one or more batteries, or even manual power (*e.g.*, a hand operated vacuum pump). In some examples, the pump 16 may be sized and shaped to fit outside of, on, or within the fluid collection device 12. For example, the pump 16 may include one or more miniaturized pumps or one or more micro pumps. The vacuum sources disclosed herein may include one or more of a switch, a button, a plug, a remote, or any other device suitable to activate the pump 16.

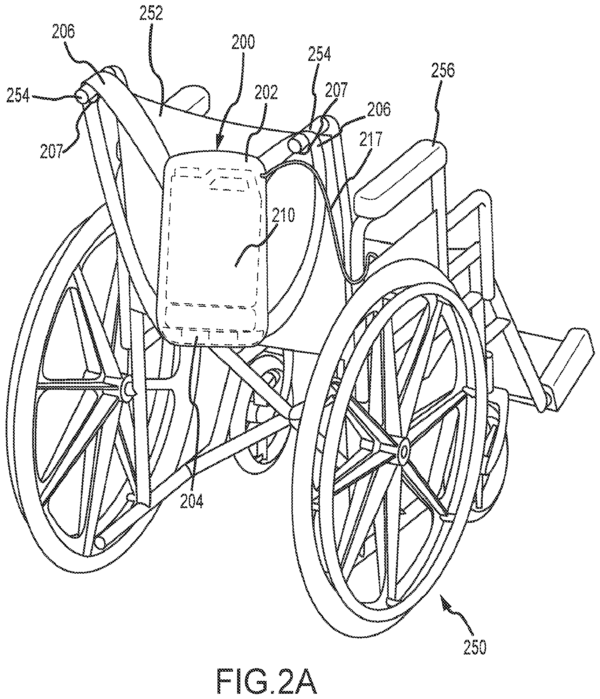

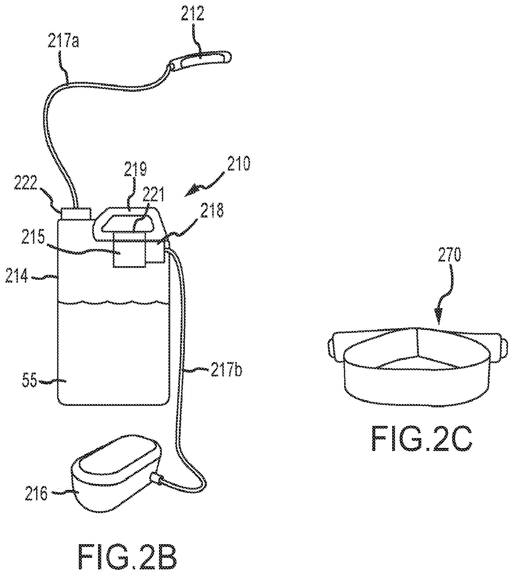

At least one, some, or all of the embodiments of urine collection systems described herein are configured to be worn by a user, positioned on a surface such as a table, and/or securable or mountable to a wheelchair. Turning to **FIG. 2A****,** a urine collection system 200 is shown secured or mounted to a wheelchair 250. The urine collection system 200 may be mounted to the wheelchair 250 with other supports not shown in **FIG. 2A****,** such as shelves, brackets, pouches, slings, and so forth. The urine collection system 200 also may be worn by a user and/or caregiver. Whether mounted to a wheelchair 250 or worn by a user, the configuration of the urine collection system 200 results in the technical effect of allowing a user to discretely use and/or transport the urine collection system 200 with the cover 202 that holds a urine storage system 210 therein. For example the cover 202 may be sized and dimensioned to hold at least a urine collection container 214, pump 216, and a sensor 215 (shown in **FIG. 2B**) therein.

The wheelchair 250 may include any of a number of different conventional wheelchairs, and may include a back 252, two handles 254, and two arms 256. The urine collection system 200 may include the cover 202 or container support that is configured to detachably secure, mount, or hang from the wheelchair 250 and support a storage system 210 of the urine collection system 200. In some embodiments, the cover 202 includes a pack or bag having one or more straps 206 configured to hang from or secure to one or more handles 254 of the wheelchair 250. The straps 206 may include one or more fasteners configured to adjustably secure the straps 206 to the wheelchair 250, such as at least one of buckles, clips, and/or hook and loop fastener materials. The straps 206 may each include a ring 207 sized and dimensioned to insert the handle 254 of the wheelchair 250 therethrough or other suitable device. The ring 207 may be positioned on an inner surface of the strap 206, as shown in **FIG. 2B****,** or an outer surface of the strap 206 such that the strap 206 does not hang over the handle 254. The ring 207 may be movably secured to the strap 206. The straps 206 may be dimensioned to allow a user or caregiver to adjust the straps 206 to move the cover 202 closer to the ground. By moving the cover 202 below the level of the seat of the wheelchair, pulling urine from the urine collection device 212 and into the urine collection container 214 may be assisted by gravity. In some embodiments, the cover 202 is configured to be worn as a backpack using the one or more straps 206.

The pack or bag of the cover 202 is sized and dimensioned to hold at least the urine collection container 214 therein. The pack of the cover 202 also may include a sleeve sized and dimensioned to hold the urine collection container 214 therein. The sleeve of the cover 202 may be insulated, such as with one or more foam members 204. The one or more foam members 204 positioned in the cover 202 result in the technical effect of reducing operational sounds of the urine collection system 200 (*e.g.* a pump 216, shown in **FIG. 2B**). In addition to abating sound, the one or more foam members may provide the technical effect of minimizing operational vibrations of the urine collection system 200. The one or more foam members 204 may be positioned underneath and/or around at least a portion of the pump 216 to minimize vibrations and sounds emanating from the pump 216. In some embodiments, the one or more foam members 204 may be positioned in one or more sleeves in an interior region of the cover 202. The pack of the cover 202 may include a reinforced base that keeps the pack upright to provide the technical effect of preventing fluid ingress into the pump 216. In some embodiments, the pack of the cover 202 may include a heat sink positioned below the pump 216 to inhibit the pump 216 from overheating. The pack of the cover 202 may include one or more air vents on the side or front of the pack for pulling air into the pack.

Turning to **FIG. 2B**, which shows a urine collection system 200 removed from the cover 202, according to an embodiment. The urine collection system 200 may include a urine collection device 212, a conduit 217, a storage system 210, and a pump 216. The urine collection device 212 is configured to be positioned at least proximate to a urethra of a user. While the urine collection device 212 shown in **FIG. 2B** includes a female urine collection device, the urine collection device 212 may instead include a male urine collection device. PCT International Application No. PCT/US2019/029616, for example, describes various embodiments of both male and female fluid collection devices that may be used in any of the embodiments disclosed herein. Moreover, the urine collection device 212 may be interchangeable in the urine collection system 200 between different types, varieties, and sizes of male or female urine collection devices. Generally, the urine collection device 212 may include a surface sized to be positioned proximate or adjacent to the urethra and configured to wick urine or other fluids away from the user. Urine or other fluids may be wicked from the surface to a reservoir in the urine collection device 212.

The urine collection system 200 also includes a first conduit 217a in fluid communication with an interior region (*e.g*. reservoir) of the urine collection device 212 and an interior region of the urine collection container 214. The first conduit 217a may be positioned between the urine collection device 212 and the urine collection container 214. The urine collection system 200 also may include a second conduit 217b providing fluid communication between the pump 216 and the interior region of the urine collection container 214. In some embodiments, the pump 216 may be secured directly to the urine collection container 214, and the second conduit 217b may be absent from the urine collection system 200. The conduits 217a, 217b may include a flexible tube. In some embodiments, at least a portion of the first conduit 217a is substantially opaque, thereby inhibiting viewing of the urine within the first conduit 217a.

The urine collection system 200 also includes a storage system 210. The storage system 210 may include the urine collection container 214 having an interior region that stores urine 55 received from the urine collection device 212 via the first conduit 217a. The urine collection container 214 may be opaque or clear according to different embodiments and may include a generally rectangular front or rear profile. In some embodiments, the urine collection container 214 includes at least one of a handle 219 or a spout 222. Urine 55 collected in the urine collection container 214 may be emptied through the spout 222 after removing a cap or cover. The urine collection container 214 may be reusable and dishwasher safe, and may include a generally rigid material such as polycarbonate, plastic, rubber, metal, glass, combinations thereof, or any other suitable materials. The urine collection container 214 may be sized and dimensioned to fit within an insulated sleeve in the pack of the cover 202.

The storage system 210 also may include a filter 218, a controller 221, and a sensor 215. The sensor 215 is secured to the urine collection container 214 and configured to detect a property relating at least to a volume of the urine 55 in the interior region of the urine collection container 214. In some embodiments, the sensor 215 may be located or positioned at an inlet (either interior or exterior) of the urine collection container 214 for the conduit 217a. The sensor 215 may be positioned along and/or within the conduit 217a, according to an embodiment. In some embodiments, the sensor 215 includes an ultrasonic sensor, a laser sensor, or an ultraviolet (UV) sensor configured to provide a continuous or periodical feedback of the property relating at least to a volume of the urine 55 without the sensor touching the urine 55. The sensor 215 may include a non-contact fluid sensor such as a capacitive sensor, an inductive sensor, a gravimetric sensor, or a mechanical float. The sensor 215 may be secured to the urine collection container with at least a portion of the sensor 215 inside the interior region of the urine collection container 214. In some embodiments, the sensor 215 is positioned at a top of the urine collection container 214 and pointed downwards where the urine 55 collects in the interior region of the urine collection container 214.

In some embodiments, the sensor 215 includes a level transmitter configured to detect a level of the urine 55 in the urine collection container 214. In an embodiment, the sensor 215 may include a laser level sensor that measures the distance between the laser transmitter of the sensor 215 to a surface of the urine 55 and back to a detector of the sensor 215. The elapsed time between transmitting the laser and detecting the reflected laser may be measured by the sensor 215 and a distance between the sensor 215 and the level of the urine 55 may be calculated by the sensor 215 or an associated controller 221. The laser may be transmitted through a clear window on the urine collection container 214 or at least the transmitter of the sensor 215 may be positioned within the urine collection container 214.

In some embodiments, the sensor 215 may include an ultrasonic level sensor that leverages the speed of sound to detect the property relating at least to a volume of the urine 55 in the urine collection container 214. For example, the sensor 215 may measure a distance between a transducer of the sensor 215 and a surface of the urine 55 in the urine collection container 214 using a time for the pulse to travel from the transducer to the surface of the urine 55 and back to the transducer of the sensor 215. The sensor 215 or a controller 221 associated with the sensor 215 may determine a volume of the urine 55 in the urine collection container 214 using the distance between the sensor 214 and the surface of the urine 55 in the urine collection container 214.

In some embodiments, the sensor 215 includes or is associated with an accelerometer that provides the technical effect of determining when the sensor 215 should detect the level of the urine 55 in the urine collection container 214. The accelerometer is configured to detect a substantially steady motion state of the urine 55 in the urine collection container 214 when the sensor 215 may transmit the laser or ultrasonic pulse for detecting a level of the urine 55 in the urine collection container 215. For example, the accelerometer may detect or indicate when the urine collection container 214 has been stationary for a predetermined or preselected period of time to steady the readings from the sensor 215 and improve accuracy of the readings from the sensor 215.

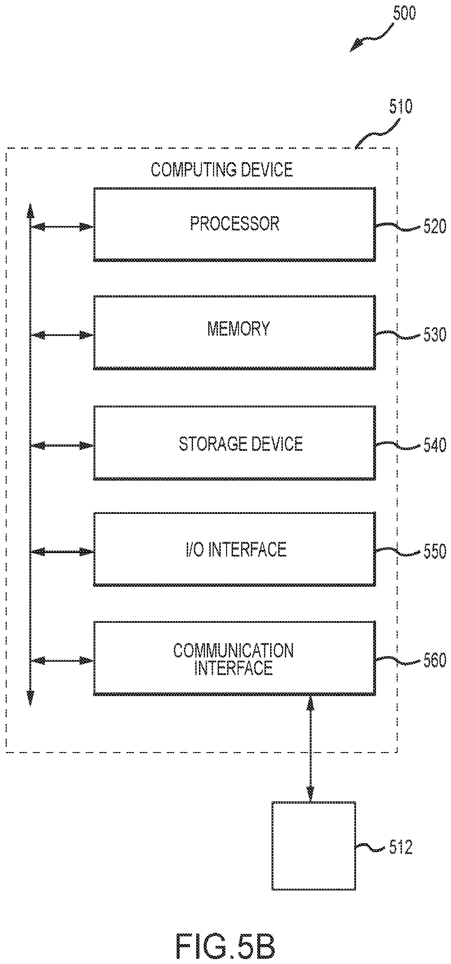

In some embodiments, the storage system 210 also includes the controller 221. The controller 221 may be configured to communicate with the sensor 215, such as a wired or wireless connection. In some embodiments, the sensor 215 may include the controller 221. The controller 221 may include a printed circuit board (PCB) equipped with erasable programmable read-only memory (EPROM) for memory of at least data collected by the sensor 215. The controller 221 may include a processor configured to calculate a level or volume of urine 55 in the urine collection container. The controller 221 may include a communication interface configured to send notifications or alerts to other electronic devices. For example, the communication interface may be configured to send notifications or alerts at a selected radio frequency, via BLUETOOTH, or via WI-FI to another electronic device, such as a mobile phone of the user or caregiver. The controller 221 may be powered by an external or internal battery, such as a rechargeable battery. **FIG. 5B** provides additional details of a controller 500 that may include the controller 221.

In some embodiments, the controller 221 provides the technical effect of wirelessly transmitting an alert to an electronic device of the user or a caregiver when the property relating at least to the volume of the urine detected by the sensor 215 indicates the volume of the urine 55 in the urine collection container 215 has reached or exceeded a predetermined volume. For example, based on data from the sensor 215, the controller 221 may wirelessly transmit an alert to an electronic device that the urine 55 in the urine collection device is a predetermined distance (such as about 2.5 cm) from the sensor 215, and emptying of the urine collection container 214 is recommended. In some embodiments, the controller 221 may wirelessly transmit alerts and selected frequencies, such as selected time and/or volume intervals. The controller 221 may wirelessly transmit an alert to the electronic device of the user or the caregiver when a battery powering at least one of the controller 221 or the pump 216 is low. The controller 221 may wirelessly transmit an alert to the electronic device of the user or the caregiver when replacement of a filter (such as the filter 218) of the urine collection system 200 is recommended.

The storage system 210 also may include the filter 218 providing the technical effect of neutralizing odor of the air being pulled from the interior region of the urine collection container 214 by the pump 216. In some embodiments, the filter 218 is positioned between at least a portion of the conduit 217b and a portion of the interior region of the urine collection container 214 such that air being pulled from the interior region of the urine collection container 214 is filtered before or as the air enters the conduit 217b. In some embodiments, the filter 218 is positioned on an exhaust vent on the pump 216. In some embodiments, a filter 218 is positioned both at the exhaust vent on the pump 216 and before air enters the conduit 217b. The filter 218 may include an odor absorbing filter and/or a hydrophobic filter configured to prevent or minimize fluid from the urine collection container 214 being pulled into the pump 216.

In some embodiments, the filter 218 may include an aromatherapy pack or an aromatherapy pack may be secured proximate to the exhaust vent of the pump resulting in the technical effect of producing a more pleasant smell. The filter may include baking soda or other composition that removes odor from the air and/or adds pleasant aroma to the air. In some embodiments, the filter 218 is absent and the pump 216 includes the aromatherapy pack removably secured to the pump 216 proximate to the exhaust vent.

The pump 216 is in fluid communication with the interior region of the urine collection container 214 and is configured to pull at least a partial vacuum on the interior region of the urine collection container 214 effective to draw the urine from the urine collection device 212 through the first conduit 217a into the urine collection container 214. The pump 216 may be secured directly to the urine collection container 214, or the conduit 217b may fluidly couple the pump 216 with the interior region of the urine collection container.

The pump 216 may include one or more of a manual vacuum pump, and electric vacuum pump, a diaphragm pump, a centrifugal pump, a displacement pump, a magnetically driven pump, a peristaltic pump, or any pump configured to produce a vacuum. For example, the pump may include an air media diaphragm pump having a minimum pumping speed of 25 ml/second. In some embodiments, the pump 216 includes a variable speed pump and/or a continuous pump. For example, the pump 216 may include a variable speed pump that operates at a low speed until the sensor 215 or other sensor detects urine passing through the conduit 217a into the urine collection container 214, when the pump 216 then adjusts to a higher speed to prevent wetting or pooling of urine at the urine collection device 212. The pump 216 may provide a vacuum or suction to remove fluid from the fluid collection device 212. In some examples, the pump 216 may be powered by one or more batteries. In some examples, the pump 216 may be sized and shaped to fit within the cover 202, such as within a sleeve within the cover 202. For example, the vacuum source 216 may include one or more miniaturized pumps or one or more micro pumps. The pump 216 may include one or more of a switch, a button, a plug, a remote, or any other device suitable to activate the pump 216.

Turning to **FIG. 2C**, the urine collection system 200 also may include one or more belts 270. The one or more belts 270 may be configured to secure the conduit 217a to the user and/or a portion of the wheelchair 250. The one or more belts 270 may include a fastener configured to allow the belt to adjust in size, such as an elastic material, hook and loop fastener material, a buckle, a clip, and so on.

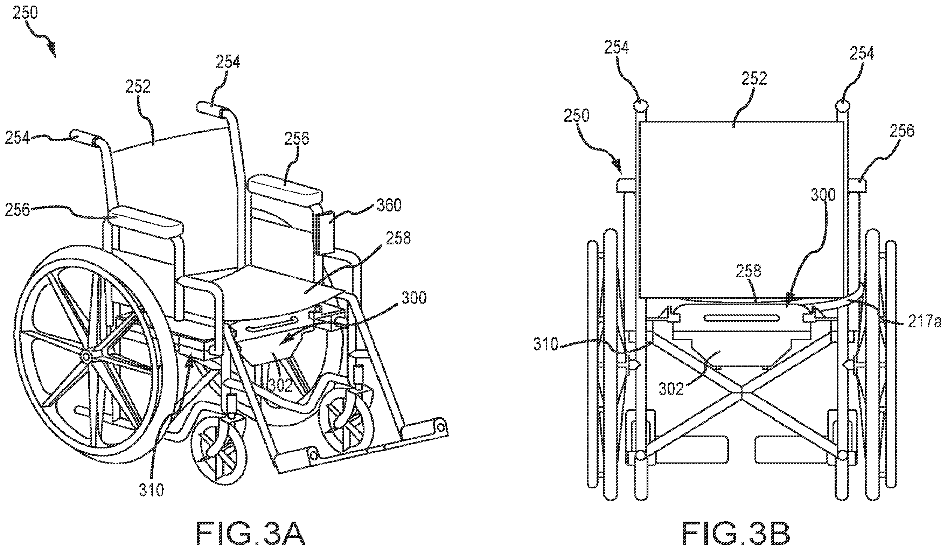

In some embodiments, the configuration of a urine collection system results in the technical effect of detachably securing or mounting the urine collection system under a seat of the wheelchair and/or rest on a flat surface. **FIGS. 3A** and **3B** show a portion of a urine collection system 300 detachably secured underneath the seat 258 of the wheelchair 250. The urine collection system 300 may include a container support 310 secured to the wheelchair 250 and a urine collection container 302. In some embodiments, the urine collection system 300 also include an electronic device 360 detachably secured or securable to the wheelchair 250. Although not shown in **FIGS. 3A** and **3B**, the urine collection system 300 also may include the urine collection device 212 described above in relation to the urine collection system 200. Unless otherwise noted, the urine collection system 300 may include any aspect of the urine collection system 200 described above. The urine collection system 300 may be mounted to the wheelchair 250 with other supports not shown in **FIG. 3A**, such as packs, shelves, brackets, pouches, slings, and so forth.

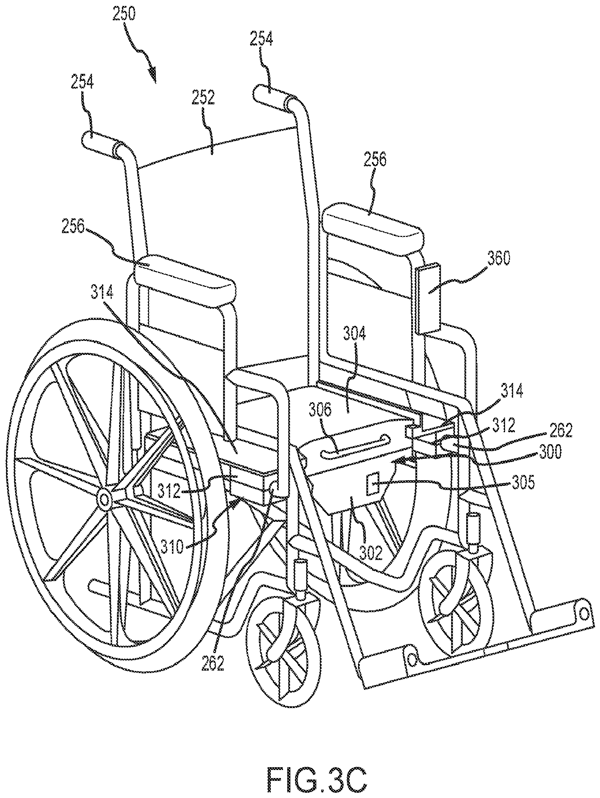

Turning in the drawings to **FIG. 3C****,** which shows the urine collection system 300 secured to the wheelchair 250 with the seat 258 removed. In some embodiments, the container support 310 includes two opposing tracks secured or securable to a frame member 262 of the wheelchair 250. For example, the container support 310 may include mounting blocks 312 mounted or mountable to the frame member 262 of the wheelchair 250 and guide brackets 314 secured or securable to the mounting blocks 312. The mounting blocks 312 and guide brackets 314 may include one or more different materials, such as steel, metal, plastic, carbon fiber, or combinations thereof. The configuration of the mounting blocks 312 and guide brackets 314 may result in the technical effect of securing the urine collection system to conventional wheelchairs as an after-market accessory.

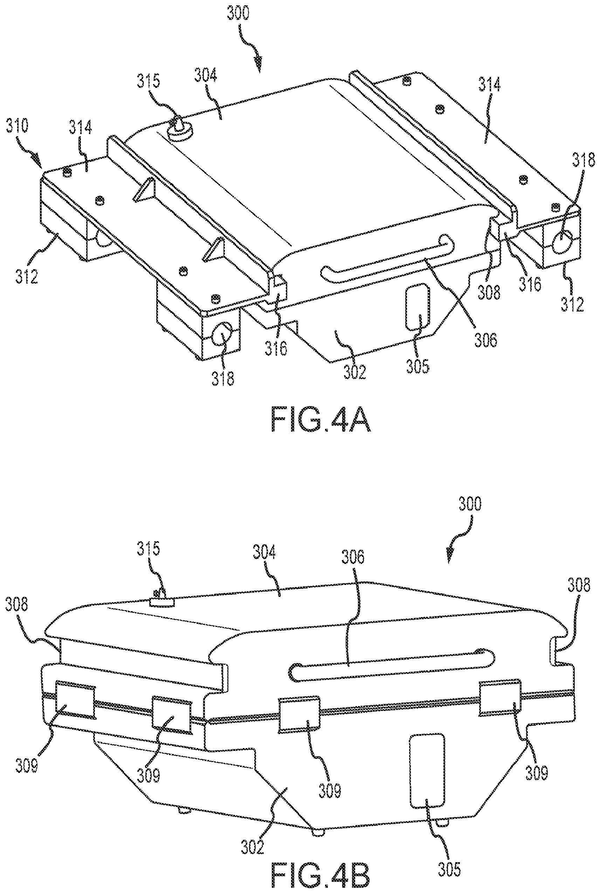

Turning to **FIGS. 4A** and **4B****,** which shows the urine collection system 300 removed from the wheelchair 250. In some embodiments, the container support 310 includes multiple mounting blocks 312, such as four mounting blocks 312. Each of the mounting blocks 312 may include a through hole 318 or bore sized to hold the frame member 262 therein. In some embodiments, the mounting blocks 312 include two members secured together around the frame member 262 to secure the mounting block 312 to the frame member 262. The guide brackets 314 may each include a track 316 protruding from the guide bracket 314. The track 316 is sized to fit at least partially within a slot 308 on one of the urine collection container 302 or the container cover 304. In some embodiments, the track 316 is sized and shaped complementary to the slot on one of the urine collection container 302 or the container cover 304. In some embodiments, the guide bracket 314 includes the slot 308 rather than the track 316, and at least one of the urine collection container 302 or the container cover 304 includes the track 316. In some embodiments, the configuration of the container support 310 resulting in the technical effect of the urine collection system 300 being adaptable to different sizes of wheelchairs. For example, the guide brackets 314 may be adjustably secured to the mounting blocks 312 to allow a user to adjust a distance between the guide brackets 314 to fit the urine collection container 302 and the container cover 304 between the guide brackets 314 and also mount the urine collection container 302 and the container cover 304 to the guide brackets 314.

The urine collection system 300 also includes the urine collection container 302 and the container cover 304, according to an embodiment. As noted above, at least one of the urine collection container 302 or the container cover 304 includes two opposing slots 308 each configured to receive a track 316 of the guide brackets 314. In the urine collection system 300 shown in **FIG. 4A**, the slots 308 are disposed on the container cover 304. In some embodiments, the slot 308 is shaped generally complementary to the track 316, resulting in the technical effect of allowing a user to slide the urine collection container 302 and the container cover 304 off and on the tracks 316.

At least one of the container cover 304 or the urine collection container 302 may include a handle 306, such as the handle 306 shown on the container cover 304 in **FIGS. 4A** and **4B****.** In many embodiments, the urine collection system 300 may include a handle 306 at both the front and the back of at least one of the container cover 304 or the urine collection container 302. The container cover 304 also may include a port 315 configured to connect or secure to the conduit 217a. The port 315 provides fluid communication between the conduit 217a and an interior region of the urine collection container 302. In some embodiments, the port 315 is sized and dimensioned to connect or secure to an adapter configured to connect to the conduit 217a. In some embodiments, at least one of the port 315, the adapter, or the conduit 217a may be barbed to more securely fit the conduit 217a and the port 315 or the adapter together. The port 315 may be positioned on a top portion of the container cover 304, according to an embodiment.

Turning specifically to **FIG. 4B****,** one or more locks 309 may provide the technical effect of detachably securing the urine collection container 302 to the container cover 304. The locks 309 may include press fit locks, snap fit locks, spring locks, latches, or other locks configured to detachably secure the urine collection container 302 to the container cover 304. The urine collection container 302 also may include a window 305 positioned to provide the technical effect of allowing a user or caregiver to view an amount of urine held in the urine collection container 304.

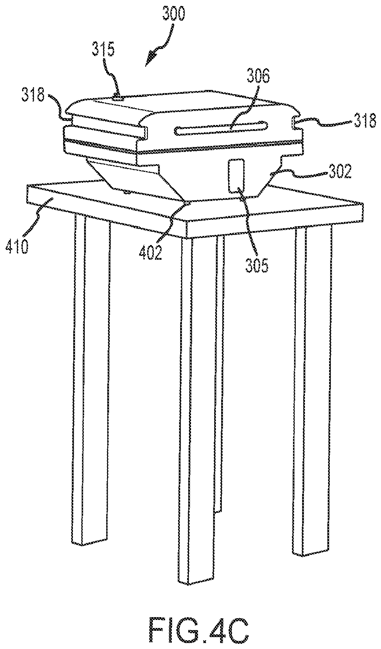

Turning to **FIG. 4C****,** in some embodiments, the configuration of the urine collection assembly 300 results in the technical effect of the urine collection assembly being used without a wheelchair (in addition to use with a wheelchair). For example, the urine collection container 302 and the container cover 304 may be positioned on other surfaces, such as a table 410, nightstand, bedside table, and so on. The urine collection container 302 may include rubber or felt feet 402 positioned on the bottom of the urine collection container 302. The urine collection container 302 may include a generally planar or flat bottom that allows the urine collection container 302 and the container cover 304 to rest on a flat surface such as the table 410. In some embodiments, the urine collection container 302 may include a curved or non-planar bottom, and the feet 402 are sized and positioned on the bottom of the urine collection container 302 to hold and stabilize the urine collection container 302 and the 304 on a flat surface.

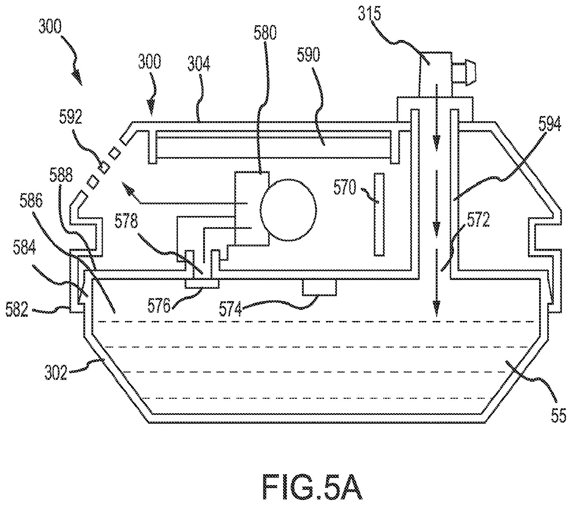

Turning to **FIG. 5A****,** which shows a cross-sectional view of the urine collection container 302 and the container cover 304. In some embodiments, the urine collection container 302 includes one or more protrusions 584 and the container cover 304 includes one or more tabs 582 positioned to engage with the one or more protrusions 584 to form a snap joint to detachably secure the container cover 304 to the urine collection container 302. The urine collection container 302 may include an interior region 586 or chamber sized to receive and hold therein the urine 55 received from the urine collection device 212. In some embodiments, the urine collection container 302 may hold about 1 liter to about 3 liters, about 1 liter, about 2 liters, or about 3 liters of the urine 55 therein. The interior region 586 of the of the urine collection container 302 may be generally enclosed. For example, the urine collection container 302 may include a basin to hold the urine 55 and a top 588 that prevents the urine in the urine collection container 302 from spilling to splashing on elements housed in the container cover 304.

The urine collection container 302 also includes an inlet 572 and an outlet 578. At least one (*e.g.* both) of the inlet 572 or the outlet 578 may extend through the top 588 of the urine collection container 302. In some embodiments, the urine collection container 302 includes a tube 594, conduit, or other element defining at least a portion of the inlet 572 and/or providing fluid communication between the port 315 and the interior region 586 of the urine collection container 302. Accordingly, the tube 594 may extend through an interior of the container cover 304 between ) the top 588 of the urine collection container 302 and the top of the container cover 304. In some embodiments, the tube 594 may be secured or integrally formed with the top 588 of the urine collection container 302. In some embodiments, the tube 594 extends through an opening in the container cover and an adapter may secure directly to the tube 594 effective to provide fluid communication between the conduit 217a secured to the adapter and the inlet 572 of the urine collection container 302.

In some embodiments, the outlet 578 extends through the top 588 of the urine collection container 304. The urine collection device 302 may include a tube, conduit, adapter, or other element configured to provide fluid communication between the pump 580 and the interior region 586 of the urine collection container 302. In some embodiments, the pump 580 is secured or mounted directly to the urine collection container with the pump in fluid communication with the outlet 578. In some embodiments, a conduit extends at least partially between the outlet 578 and the pump 580 to provide fluid communication therebetween.

The urine collection system 300 also may include a filter 576. The filter 576 may cover the outlet 576 or be positioned within the outlet between the pump 580 and the interior region 586 of the urine collection container 302. The configuration and positioning of the filter 576 may provide the technical effect of preventing urine 55 in the urine collection container 302 from passing through the outlet 576 and/or remove at least some odor of the air being pulled from the urine collection container 302 by the pump 580. The filter 576 may include any aspect of the filter 218 of the urine collection system 200 described above. For example, the filter 576 may include a hydrophobic filter that prevents the urine 55 from entering the pump 580.

The urine collection system 300 also may include a sensor 574 secured or securable to the urine collection container 302. In some embodiments, the sensor 574 may be secured within the interior region 586 of the urine collection container 302. In some embodiments, the sensor 574 may be secured outside the interior region 586 of the urine collection container 302 and the urine collection container may include a window positioned to allow the sensor 574 to detect the urine 55 in the urine collection container 302. The sensor 574 may include any aspect of the sensor 215 described above, such as an ultrasonic sensor configured to provide continuous feedback of a level of the urine 55 in the urine collection container 302.

The urine collection system 300 also may include the pump 580, a battery 590, and a controller 570. At least one (*e.g.,* all) of the pump 580, the battery 590, and the controller 570 may 30 be housed in an interior region or enclosure defined at least partially by the container cover 304 and may be held in place in the interior region of the container cover 304 using a molded foam, such as a molded polyurethane foam. The pump 580 is in fluid communication with the interior region 586 of the urine collection container 302 or may be positioned to be in fluid communication with the interior region 586 of the urine collection container 302. As noted above, the pump 580 may be secured directly to the urine collection container 302, or a conduit or adapted may be fitted between the outlet and the pump 580.

The pump 580 may include any aspect of the pump 216 of the urine collection system 200 described above. For example, the pump 580 may include a diaphragm pump. When activated, the pump 580 creates a suction in the interior region 586 of the urine collection container 302 effective to pull urine from the urine collection device 212, through the conduit 217a and the tube 594 and into the interior region 586 of the urine collection container 302. The container cover 592 also may include an exhaust vent 592 that allows air to exit the interior region of the container cover 304. In some embodiments, at least one of a filter, an odor neutralizer, or an aromatherapy device or cartridge may be positioned proximate to or covering the exhaust vent 592.

The battery 590 may be electrically coupled to at least one (*e.g.,* all) of the pump 580, the sensor 574, or the controller 570 and configured to provide power to at least one (*e.g.,* all) of the pump 580, the sensor 574, and the controller 570. In some embodiments, the battery 590 may include a lithium ion battery. The controller 570 may include a PCB having control circuitry configured to activate and deactivate the pump 580. The PCB of the controller 570 also may include a battery recharged circuit electrically coupled to the battery 590 and a sensor circuit electrically coupled to the sensor 574. The controller 570 may include any aspect of the controller 221 of the urine collection system 200 described above. The controller 570 may include a processor configured to calculate a level or volume of urine 55 in the urine collection container 302. The controller 570 may include a communication interface configured to send notifications or alerts to other electronic devices. For example, the communication interface may be configured to send notifications or alerts at a selected radio frequency, via BLUETOOH, or via WI-FI to another electronic device, such as the electronic device 360 and/or a mobile phone of the user or caregiver. **FIG. 5B** provides additional details of a controller 500 that may include the controller 570.

In operation, activation of the pump 580 may pull an at least partial vacuum in the interior region 586 of the urine collection container 302, which in turn pulls an at least partial vacuum on the urine collection device 212 via the conduit 217a and the inlet 572. The vacuum pulled by the pump 580 pulls urine in the urine collection device 212 through the conduit 217a and the inlet 572 into the interior region 586 of the urine collection container 302 for temporary storage. The sensor 574 may take periodic or continuous readings of the property relating to the volume of the urine 55 in the interior region 586 of urine collection container 302. When the controller 570 determines the volume of the urine 55 in the interior region 586 of the urine collection container 302 has met or exceeded a predetermined threshold (such as 75% or 90% of the total volume of the interior region 586) base on the reading from the sensor 574, the controller 570 may transmit an electronic alert to the electronic device 360. Upon receipt of the electronic alert from the controller 570, the electronic device 360 may vibrate or beep, suggesting to the user or caregiver that the urine collection container 302 be emptied. The user or caregiver may then disconnect the conduit 217a from the port 315, and remove the urine collection container 302 and the container cover 304 from the container support 310. The container cover 304 may be removed from the urine collection container 302, and the user or caregiver may empty the urine 55 from the urine collection container 302 through the tube 594, which may function as a pour spout.

**FIG. 5B** is a schematic of a controller 500 that may be used with any of the systems and methods described herein, according to an embodiment. For example, the controller 221 and/or the controller 570 may include any aspect of the controller 500. The controller 500 may be configured to implement any of the example acts or steps disclosed herein, such as wired or wireless communication with the sensor 215, the sensor 570, the electronic device 360, other electronic devices, and/or determining a volume of the urine 55 in the urine collection container 214, 302.

The controller 500 includes at least one computing device 510, according to an embodiment. The at least one computing device 510 is an exemplary computing device that may be configured to perform one or more of the acts described above. The computing device 510 can comprise at least one processor 520, memory 530, a storage device 540, an input/output ("I/O") device/interface 550, and a communication interface 560. While an example computing device 510 is shown in **FIG. 5B****,** the components illustrated in **FIG. 5B** are not intended to be limiting of the controller 500 or computing device 510. Additional or alternative components may be usedin some examples. Further, in some examples, the controller 500 or the computing device 510 can include fewer components than those shown in **FIG. 5B****.** For example, the controller 500 may not include the one or more additional computing devices 512. In some examples, the at least one computing device 510 may include a plurality of computing devices. Components of computing device 510 shown in **FIG. 5B** are described in additional detail below.

In some examples, the processor(s) 520 includes hardware for executing instructions (e.g., instructions for carrying out one or more portions of any of the methods disclosed herein), such as those making up a computer program. For example, to execute instructions, the processor(s) 520 may retrieve (or fetch) the instructions from an internal register, an internal cache, the memory 530, or a storage device 540 and decode and execute them. In particular examples, processor(s) 520 may include one or more internal caches for data such as tables pertaining to volumetric tables. As an example, the processor(s) 520 may include one or more instruction caches, one or more data caches, and one or more translation lookaside buffers (TLBs). Instructions in the instruction caches may be copies of instructions in memory 530 or storage device 540. In some examples, the processor 520 may be configured (e.g., include programming stored thereon or executed thereby) to carry out one or more portions of any of the example methods disclosed herein.

In some examples, the processor 520 is configured to perform any of the acts disclosed herein or cause one or more portions of the computing device 510 or controller 500 to perform at least one of the acts disclosed herein. Such configuration can include one or more operational programs (e.g., computer program products) that are executable by the at least one processor 520. For example, the processor 520 may be configured to automatically determine a volume of urine in a urine collection container, automatically determine a proximity of urine in the urine collection container to a sensor, automatically transmit an alert when the volume of the urine in the urine collection container meets or exceeds a predetermined threshold, automatically transmit an alert when a change of filter is suggested, and/or automatically transmit an alert when a change or recharge of battery is suggested.

The at least one computing device 510 (e.g., a server) may include at least one memory storage medium (e.g., memory 530 and/or storage device 540). The computing device 510 may include memory 530, which is operably coupled to the processor(s) 520. The memory 530 may be used for storing data, metadata, and programs for execution by the processor(s) 520. The memory 530 may include one or more of volatile and non-volatile memories, such as Random Access Memory (RAM), Read Only Memory (ROM), a solid state disk (SSD), Flash, Phase Change Memory (PCM), or other types of data storage. The memory 530 may be internal or distributed memory.

The computing device 510 may include the storage device 540 having storage for storing data or instructions. The storage device 540 may be operably coupled to the at least one processor 520. In some examples, the storage device 540 can comprise a non-transitory memory storage medium, such as any of those described above. The storage device 540 (e.g., non-transitory storage medium) may include a hard disk drive (HDD), a floppy disk drive, flash memory, an optical disc, a magneto-optical disc, magnetic tape, or a Universal Serial Bus (USB) drive or a combination of two or more of these. Storage device 540 may include removable or non-removable (or fixed) media. Storage device 540 may be internal or external to the computing device 510. In some examples, storage device 540 may include non-volatile, solid-state memory. In some examples, storage device 540 may include read-only memory (ROM). Where appropriate, this ROM may be mask programmed ROM, programmable ROM (PROM), erasable PROM (EPROM), electrically erasable PROM (EEPROM), electrically alterable ROM (EAROM), or flash memory or a combination of two or more of these. In some examples, one or more portions of the memory 530 and/or storage device 540 (e.g., memory storage medium(s)) may store one or more databases thereon.

In some examples, one or more of a history of the volume of the urine in the urine collection container, a trend of the volume of the urine in the urine collection container, a history of filter replacement, and/or a history of battery replacement or recharging may be stored in a memory storage medium such as one or more of the at least one processor 520 (e.g., internal cache of the processor), memory 530, or the storage device 540. In some examples, the at least one processor 520 may be configured to access (e.g., via bus 570) the memory storage medium(s) such as one or more of the memory 530 or the storage device 540. For example, the at least one processor 520 may receive and store the data (e.g., look-up tables) as a plurality of data points in the memory storage medium(s). The at least one processor 520 may execute programming stored therein adapted access the data in the memory storage medium(s) to automatically determine a volume of urine in a urine collection container, automatically determine a proximity of urine in the urine collection container to a sensor, automatically transmit an alert when the volume of the urine in the urine collection container meets or exceeds a predetermined threshold, automatically transmit an alert when a change of filter is suggested, and/or automatically transmit an alert when a change or recharge of battery is suggested. For example, the at least one processor 520 may access one or more look-up tables in the memory storage medium(s) such as memory 530 or storage device 540.

The computing device 510 also includes one or more I/O devices/interfaces 550, which are provided to allow a user to provide input to, receive output from, and otherwise transfer data to and from the computing device 510. These I/O devices/interfaces 550 may include a mouse, keypad or a keyboard, a touch screen, camera, optical scanner, network interface, web-based access, modem, a port, other known I/O devices or a combination of such I/O devices/interfaces 550. The touch screen may be activated with a stylus or a finger.

The I/O devices/interfaces 550 may include one or more devices for presenting output to a user, including, but not limited to, a graphics engine, a display (e.g., a display screen or monitor), one or more output drivers (e.g., display drivers), one or more audio speakers, and one or more audio drivers. In certain examples, I/O devices/interfaces 550 are configured to provide graphical data to a display for presentation to a user. The graphical data may be representative of one or more graphical user interfaces and/or any other graphical content as may serve a particular implementation.

The computing device 510 can further include a communication interface 560. The communication interface 560 can include hardware, software, or both. The communication interface 560 can provide one or more interfaces for communication (such as, for example, packet-based communication) between the computing device 510 and one or more additional computing devices 512 or one or more networks. For example, communication interface 560 may include a network interface controller (NIC) or network adapter for communicating with an Ethernet or other wire-based network or a wireless NIC (WNIC) or wireless adapter for communicating with a wireless network, such as a WI-FI. The one or more additional computer device 512 may include the electronic device 360, a smart phone of the user, a smart phone of the caregiver, and/or a computer device of a healthcare system.

Any suitable network and any suitable communication interface 560 may be used. For example, computing device 510 may communicate with an ad hoc network, a personal area network (PAN), a local area network (LAN), a wide area network (WAN), a metropolitan area network (MAN), or one or more portions of the Internet or a combination of two or more of these. One or more portions of one or more of these networks may be wired or wireless. As anexample, one or more portions of controller 500 or computing device 510 may communicate with a wireless PAN (WPAN) (such as, for example, a BLUETOOTH WPAN), a WI-FI network, a WI-MAX network, a cellular telephone network (such as, for example, a Global System for Mobile Communications (GSM) network), or other suitable wireless network or a combination thereof. The computing device 510 may include any suitable communication interface 560 for any of these networks, where appropriate.

The computing device 510 may include a bus 570. The bus 570 can include hardware, software, or both that couples components of computing device 510 to each other. For example, bus 570 may include an Accelerated Graphics Port (AGP) or other graphics bus, an Enhanced Industry Standard Architecture (EISA) bus, a front-side bus (FSB), a HYPERTRANSPORT (HT) interconnect, an Industry Standard Architecture (ISA) bus, an INFINIBAND interconnect, a low-pin-count (LPC) bus, a memory bus, a Micro Channel Architecture (MCA) bus, a Peripheral Component Interconnect (PCI) bus, a PCI-Express (PCIe) bus, a serial advanced technology attachment (SATA) bus, a Video Electronics Standards Association local (VLB) bus, or another suitable bus or a combination thereof.

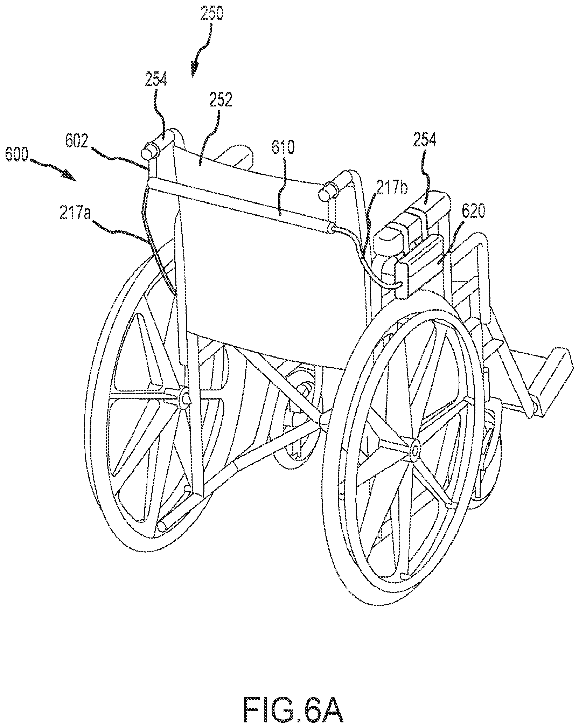

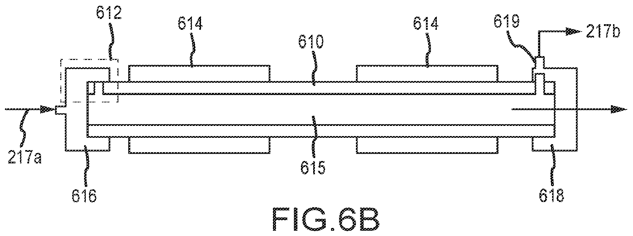

Turning ahead in the drawings to **FIGS. 6A** and **6B****,** in some embodiments of a urine collection system 600, the container support includes two arms 602 configured to detachably mount or hang from handles 254 of the wheelchair 250 and the urine collection container includes a hollow bar 610 secured to the two arms 602 and having the interior region 615. The urine collection system 600 may be mounted or mountable to the wheelchair 250 with other supports not shown in **FIG. 6A****,** such as shelves, brackets, pouches, slings, and so forth. The hollow tube 610 may be generally rigid, or more rigid that urine collection device 212 and/or the conduit 217a. In some embodiments, the urine collection system 600 includes grips 614, such as polyurethane grips secured or securable to the hollow tube 610.

The hollow tube 610 may include a diameter of about 25 mm to about 75 mm, about 30 mm to about 40 mm, about 40 mm to about 50 mm, about 45 to about 55 mm, about 50 mm to about 60 mm, about 60 to about 70 mm, about 30 mm, about 40 mm, about 50 mm, about 60 mm, or about 70 mm. The hollow tube 610 may include a length of about 30 cm to about 90 cm, about 30 to about 50 cm, about 50 cm to about 70 cm, about 70 to about 90 cm, about 30 cm, about 40 cm, about 50 cm, about 60 cm, about 70 cm, about 80 cm, or about 90 cm. The interior region 615 of the hollow tube 610 may be sized to hold about 0.5 L to about 2 L of urine, about 0.5 L to about 1 L of urine, about 1 L to about 1.5 L of urine, about 1.5 L to about 2 L of urine, about 0.5 L of urine, about 0.75 L of urine, about 1 L of urine, about 1.25 L of urine, about 1.5 L of urine, about 1.75 L of urine, or about 2 L of urine.

The conduit 217a provides fluid communication between the interior region 615 of the hollow bar 610 and the urine collection device 212 (not shown). In some embodiments, the urine collection system 600 includes a port or adapter 616 secured or securable to the hollow bar 610. The adapter 616 is configured to secure to the conduit 217a effective to provide fluid communication between the conduit 217a and the interior region 615 of the hollow tube 610. The urine collection system also may include a buzzer 612 or other alert device and/or a sensor (not shown). The sensor may be configured to detect a property related at least to a volume of the urine in the hollow tube 610 and may include any aspect of the sensors 215, 574 described above. In some embodiments, the buzzer 612 is electrically coupled to sensor and configured to buzz, vibrate, or ring when the fluid detected by the sensor meets or exceeds a threshold described above in relation to urine collection systems 200, 300.

In some embodiments, the urine collection system 600 includes a port or adapter 618 secured or securable to the hollow bar 610. The port or adapter 618 may be positioned generally distal or opposite to the port or adapter 616, *e.g.*, the port or adapter 618 and the port or adapter 616 may be positioned at opposite ends of the hollow bar 610. The port or adapter 618 is configured to provide fluid communication between a pump (not shown) and the interior region
615 of the hollow bar 610. In some embodiments, the pump may be secured directly to the port or adapter 618. In some embodiments, the conduit 217b provides fluid communication between the pump and the interior region 615 of the hollow bar 610. The urine collection system 600 also may include the pump (not shown) having any aspect of the pumps 216, 580 described above.

In some embodiments, the urine collection system 600 also includes a cover such as a bag 620 or case configured to detachably secure to an arm 254 of the wheelchair 250. The bag 620 is sized and dimensioned to house at least the pump therein. In some embodiments, the urine collection system 600 also includes a controller and/or a battery (not shown), and the bag 620 is sized and dimensioned to house at least the pump, the battery, and controller. The controller and/or the battery of the urine collection system 600 may include any aspect of the controllers 221, 500, 570 and the battery 590 described above.

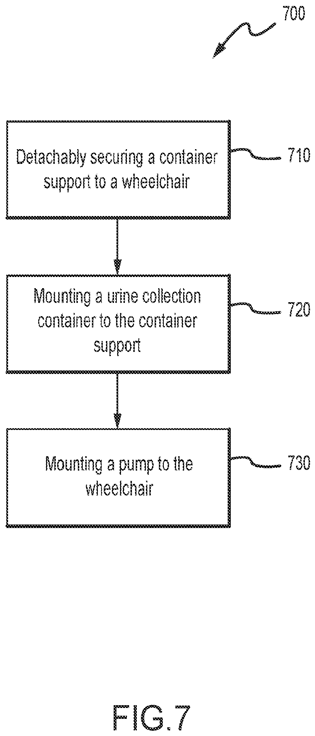

**FIG. 7** is a flow diagram of a method 700 for assembling a portable urine collection system, according to an embodiment. The method 700 includes an act 710 of detachably securing a container support to a wheelchair. The method 700 also includes an act 720 of mounting a urine collection container to the container support. The urine collection container may include a sensor secured thereto and configured to detect a property relating at least to a volume of the urine in the urine collection container. In some embodiments, the method 700 also may include positioning a urine collection device at least proximate to a urethra of a user and fluidly coupling the urine collection device to the urine collection container with a first conduit. The method also includes an act 730 of mounting a pump to the wheelchair. The act 730 may include mounting a pump to the wheelchair with the pump in fluid communication with the urine collection container and configured to pull a vacuum on the interior region of the urine collection container effective to draw urine from the urine collection device through the first conduit into the urine collection container.

The method 700 may include assembling any of the urine collection systems described herein. For example, the act 710 of detachably securing a container support to the wheelchair may include securing one or more straps of a pack to one or more handles of the wheelchair. In some embodiments, the act 720 of mounting a urine collection container to the container support includes positioning the urine collection container inside the pack to mount the urine collection container. The method 700 also may include an act of positioning the urine collection container inside the pack to mount the urine collection container includes positioning the urine collection container inside a sleeve inside the pack. In some embodiments, the method 700 further includes an act of fluidly coupling the urine collection container to the pump with a second conduit and/or securing the first conduit to a user or the wheelchair with a belt.

In some embodiments, the act 710 of detachably securing a container support to a wheelchair may include securing two tracks of the container support to the wheelchair. The act 720 of mounting a urine collection container to the container support may include sliding the two tracks into two opposing slots on the urine collection container.

The act 710 of detachably securing a container support to a wheelchair may include mounting or hanging two arms of the container support from handles of the wheelchair. The act 30 720 of mounting a urine collection container to the container support may include securing a hollow bar of the urine collection container to the two arms of the container support. In some embodiments, the act 730 of mounting a pump to the wheelchair may include detachably securing a bag housing the pump to an arm of the wheelchair.

The acts of the method 700 described above are for illustrative purposes. For example, the acts of the method 700 can be performed in different orders, split into multiple acts, modified, supplemented, or combined. In an embodiment, one or more of the act of the method 700 can be omitted from the method 700. Any of the acts of the method 700 can include using any of the portable urine collection systems disclosed herein.

As used herein, the term "about" or "substantially" refers to an allowable variance of the term modified by "about" or "substantially" by ±10% or ±5%. Further, the terms "less than," "or less," "greater than," "more than," or "or more" include, as an endpoint, the value that is modified by the terms "less than," "or less," "greater than," "more than," or "or more."

While various aspects and embodiments have been disclosed herein, other aspects and embodiments are contemplated. The various aspects and embodiments disclosed herein are for purposes of illustration and are not intended to be limiting.

## Claims

1. A portable urine collection system (300), comprising:
a urine collection device (212) configured to be positioned at least proximate to a urethra of a user;
a first conduit (217a) in fluid communication with the urine collection device (212);
a urine collection container (302) having an interior region (586);
a pump (580) in fluid communication with the urine collection container (302) and configured to pull a vacuum on the interior region (586) of the urine collection container (302) effective to draw urine (55) from the urine collection device (212) through the first conduit (217a) into the urine collection container (302);
a sensor (574) secured or securable to the container and configured to detect a property relating at least to a volume of the urine in the urine collection container (302); and
a cover configured to hold at least the pump (580) therein and obscure at least the pump (580) from view outside the cover, wherein the cover includes a container cover (304), the urine collection container (302) having a generally planar bottom such that the urine collection container (302) and the container cover (304) are generally stable on a flat surface;
**characterized in that** the container cover (304) is configured to detachably secure to the urine collection container (302) and **in that** the portable urine collection system further comprises a container support (310) configured to detachably mount to a wheelchair, the container support (310) having two opposing tracks (316),
wherein at least one of the container cover (304) or the urine collection container (302) includes opposing slots (308) sized to receive the two opposing tracks (316) therein effective to detachably mount the container cover (304) and the urine collection container (302) to the two opposing tracks (316).

2. The portable urine collection system (300) of claim 1, further comprising a hydrophobic filter positioned between the urine collection container (302) and the pump (580).

3. The portable urine collection system (300) of any of claims 1 or 2, further comprising a power source operably coupled to the pump (580).

4. The portable urine collection system (300) of any of claims 1-3, wherein the pump (580) includes an exhaust filter configured to at least partially filter air exhausted from the pump (580).

5. The portable urine collection system (300) of claim 4, further comprising an aromatherapy accessory secured or securable to the pump (580) proximate to the exhaust filter.

6. The portable urine collection system (300) of any of claims 1-5, further comprising a controller configured to communicate with the sensor (574) and wirelessly transmit an alert to an electronic device when the property relating at least to the volume of the urine detected by the sensor (574) indicates the volume of the urine in the urine collection container (302) has reached or exceeded a predetermined volume.

7. The portable urine collection system (300) of claim 1, wherein the container cover (304) at least partially houses the pump (580), the power source, and the controller when secured to the urine collection container (302).

8. The portable urine collection system (300) of claim 7, further comprising a polyurethane foam housed in the container cover (304) and securing one or more of the pump (580), the power source, and the controller to the container cover (304).

9. The portable urine collection system (300) of any of claims 1-8, wherein the two opposing tracks (316) are configured to detachably mount to the wheelchair below a seat of the wheelchair.

10. The portable urine collection system of claim 1, wherein the container cover (304) includes the two opposing slots (308).

11. The portable urine collection system of claim 1, wherein the container support (310) includes:
two guide brackets each including one of the two opposing tracks (316); and
two or more mounting blocks, each guide bracket of the two guide brackets being secured to at least one mounting block of the two or more mounting blocks, wherein each mounting block of the two or more mounting blocks includes a through hole or bore sized to hold a portion of a wheelchair frame therein.

12. A method of assembling the portable urine collection system (300) of any of claims 1-11, the method comprising:
detachably securing the container support to a wheelchair;
sliding the two opposing tracks into two opposing slots of the urine collection assembly to mount the urine collection assembly to the container support secured to the wheelchair;
positioning a urine collection device proximate to a urethra of a user; and
fluidly coupling the urine collection device to the urine collection container with a first conduit.

## Patentansprüche

1. Tragbares Urinsammelsystem (300), umfassend:
eine Urinsammelvorrichtung (212), die dazu konfiguriert ist, mindestens in der Nähe einer Harnröhre eines Benutzers positioniert zu werden;
eine erste Leitung (217a) in Fluidkommunikation mit der Urinsammelvorrichtung (212);
einen Urinsammelbehälter (302) mit einem Innenbereich (586);
eine Pumpe (580), die in Fluidkommunikation mit dem Urinsammelbehälter (302) steht und so konfiguriert ist, dass sie einen Unterdruck auf den Innenbereich (586) des Urinsammelbehälters (302) ausübt, der bewirkt, dass Urin (55) aus der Urinsammelvorrichtung (212) durch die erste Leitung (217a) in den Urinsammelbehälter (302) gezogen wird;
einen Sensor (574), der an dem Behälter befestigt oder befestigbar ist und so konfiguriert ist, dass er eine Eigenschaft erfasst, die sich zumindest auf ein Volumen des Urins in dem Urinsammelbehälter (302) bezieht; und
einen Deckel, der so konfiguriert ist, dass er zumindest die Pumpe (580) darin hält und zumindest die Pumpe (580) von der Sicht außerhalb des Deckels verdeckt, wobei der Deckel einen Behälterdeckel (304) beinhaltet, wobei der Urinsammelbehälter (302) einen im Allgemeinen planaren Boden aufweist, sodass der Urinsammelbehälter (302) und der Behälterdeckel (304) im Allgemeinen auf einer ebenen Oberfläche stabil sind;
**dadurch gekennzeichnet, dass** der Behälterdeckel (304) so konfiguriert ist, dass er abnehmbar an dem Urinsammelbehälter (302) befestigt werden kann, und dadurch, dass das tragbare Urinsammelsystem ferner einen Behälterträger (310) umfasst, der so konfiguriert ist, dass er abnehmbar an einem Rollstuhl montiert wird, wobei der Behälterträger (310) zwei gegenüberliegende Schienen (316) aufweist,
wobei mindestens einer zwischen dem Behälterdeckel (304) oder dem Urinsammelbehälter (302) gegenüberliegende Schlitze (308) beinhaltet, die bemessen sind, um die zwei gegenüberliegenden Schienen (316) darin aufzunehmen, die bewirken, dass der Behälterdeckel (304) und der Urinsammelbehälter (302) abnehmbar an den zwei gegenüberliegenden Schienen (316) montiert werden.

2. Tragbares Urinsammelsystem (300) nach Anspruch 1, ferner umfassend einen hydrophoben Filter, der zwischen dem Urinsammelbehälter (302) und der Pumpe (580) positioniert ist.

3. Tragbares Urinsammelsystem (300) nach einem der Ansprüche 1 oder 2, ferner umfassend eine Stromquelle, die betriebswirksam mit der Pumpe (580) gekoppelt ist.

4. Tragbares Urinsammelsystem (300) nach einem der Ansprüche 1-3, wobei die Pumpe (580) einen Abluftfilter beinhaltet, der so konfiguriert ist, dass er die von der Pumpe (580) abgegebene Luft zumindest teilweise filtert.

5. Tragbares Urinsammelsystem (300) nach Anspruch 4, ferner umfassend ein Aromatherapiezubehörteil, das an der Pumpe (580) in der Nähe des Abluftfilters befestigt oder befestigbar ist.

6. Tragbares Urinsammelsystem (300) nach einem der Ansprüche 1-5, ferner umfassend eine Steuerung, die so konfiguriert ist, dass sie mit dem Sensor (574) kommuniziert und drahtlos einen Alarm an eine elektronische Vorrichtung übermittelt, wenn die Eigenschaft, die sich zumindest auf das durch den Sensor (574) erfasste Volumen des Urins bezieht, anzeigt, dass das Volumen des Urins in dem Urinsammelbehälter (302) ein vorbestimmtes Volumen erreicht oder überschritten hat.

7. Tragbares Urinsammelsystem (300) nach Anspruch 1, wobei der Behälterdeckel (304) zumindest teilweise die Pumpe (580), die Stromquelle und die Steuerung aufnimmt, wenn sie an dem Urinsammelbehälter (302) befestigt sind.

8. Tragbares Urinsammelsystem (300) nach Anspruch 7, ferner umfassend einen Polyurethanschaum, der im Behälterdeckel (304) untergebracht ist und eines oder mehrere von der Pumpe (580), der Stromquelle und der Steuerung am Behälterdeckel (304) befestigt.

9. Tragbares Urinsammelsystem (300) nach einem der Ansprüche 1-8, wobei die zwei gegenüberliegenden Schienen (316) so konfiguriert sind, dass sie abnehmbar an dem Rollstuhl unterhalb eines Sitzes des Rollstuhls montiert werden.

10. Tragbares Urinsammelsystem nach Anspruch 1, wobei der Behälterdeckel (304) die zwei gegenüberliegenden Schlitze (308) beinhaltet.

11. Tragbares Urinsammelsystem nach Anspruch 1, wobei der Behälterträger (310) Folgendes beinhaltet:
zwei Führungshalterungen, die jeweils eine der zwei gegenüberliegenden Schienen (316) beinhalten;
und
zwei oder mehr Montageblöcke, wobei jede Führungshalterung der zwei Führungshalterungen an mindestens einem Montageblock der zwei oder mehreren Montageblöcke befestigt ist, wobei jeder Montageblock der zwei oder mehreren Montageblöcke ein Durchgangsloch oder eine Bohrung beinhaltet, das/die so bemessen ist, dass ein Abschnitt eines Rollstuhlrahmens darin gehalten wird.

12. Verfahren zum Zusammenbauen des tragbaren Urinsammelsystems (300) nach einem der Ansprüche 1-11, wobei das Verfahren Folgendes umfasst:
abnehmbares Befestigen des Behälterträgers an einem Rollstuhl;
Schieben der zwei gegenüberliegenden Schienen in zwei gegenüberliegende Schlitze der Urinsammelbaugruppe, um die Urinsammelbaugruppe an dem am Rollstuhl befestigten Behälterträger zu montieren;
Positionieren einer Urinsammelvorrichtung in der Nähe einer Harnröhre eines Benutzers; und
fluidisches Ankoppeln der Urinsammelvorrichtung an den Urinsammelbehälter mit einer ersten Leitung.

## Revendications

1. Système portable de collecte d'urine (300), comprenant :
un dispositif de collecte d'urine (212) configuré pour être positionné au moins à proximité de l'urètre d'un utilisateur ;
un premier conduit (217a) en communication fluidique avec le dispositif de collecte d'urine (212) ;
un récipient de collecte d'urine (302) ayant une zone intérieure (586) ;
une pompe (580) en communication fluidique avec le récipient de collecte d'urine (302) et configurée pour tirer un vide sur la région intérieure (586) du récipient de collecte d'urine (302) afin d'aspirer l'urine (55) du dispositif de collecte d'urine (212) à travers le premier conduit (217a) dans le récipient de collecte d'urine (302) ;
un capteur (574) fixé ou pouvant être fixé au récipient et configuré pour détecter une propriété liée au moins à un volume d'urine dans le récipient de collecte d'urine (302) ; et
un couvercle configuré pour contenir au moins la pompe (580) et cacher au moins la pompe (580) de la vue à l'extérieur du couvercle, dans lequel le couvercle comprend un couvercle de récipient (304), le récipient de collecte d'urine (302) ayant un fond généralement plan de sorte que le récipient de collecte d'urine (302) et le couvercle de récipient (304) sont généralement stables sur une surface plane ;
**caractérisé en ce que** le couvercle de récipient (304) est configuré pour se fixer de manière amovible au récipient de collecte d'urine (302) et **en ce que** le système portable de collecte d'urine comprend en outre un support de récipient (310) configuré pour se fixer de manière amovible à un fauteuil roulant, le support de récipient (310) ayant deux pistes opposées (316),
dans lequel au moins l'un du couvercle de récipient (304) ou du récipient de collecte d'urine (302) comprend des fentes opposées (308) dimensionnées pour recevoir les deux pistes opposées (316) à l'intérieur, permettant de monter de manière amovible le couvercle de récipient (304) et le récipient de collecte d'urine (302) sur les deux pistes opposées (316).

2. Système portable de collecte d'urine (300) selon la revendication 1, comprenant en outre un filtre hydrophobe placé entre le récipient de collecte d'urine (302) et la pompe (580).

3. Système portable de collecte d'urine (300) selon l'une quelconque des revendications 1 ou 2, comprenant en outre une source d'énergie couplée de manière opérationnelle à la pompe (580).

4. Système portable de collecte d'urine (300) selon l'une quelconque des revendications 1 à 3, dans lequel la pompe (580) comprend un filtre d'échappement configuré pour filtrer au moins partiellement l'air évacué de la pompe (580).

5. Système portable de collecte d'urine (300) selon la revendication 4, comprenant en outre un accessoire d'aromathérapie fixé ou pouvant être fixé à la pompe (580) à proximité du filtre d'échappement.

6. Système portable de collecte d'urine (300) selon l'une quelconque des revendications 1 à 5 comprenant en outre un dispositif de commande configuré pour communiquer avec le capteur (574) et transmettre sans fil une alerte à un dispositif électronique lorsque la propriété relative au moins au volume d'urine détecté par le capteur (574) indique que le volume d'urine dans le récipient de collecte d'urine (302) a atteint ou dépassé un volume prédéterminé.

7. Système portable de collecte d'urine (300) selon la revendication 1, dans lequel le couvercle de récipient (304) loge au moins partiellement la pompe (580), la source d'énergie et le dispositif de commande lorsqu'il est fixé au récipient de collecte d'urine (302).

8. Système portable de collecte d'urine (300) selon la revendication 7, comprenant en outre une mousse de polyuréthane logée dans le couvercle de récipient (304) et fixant une ou plusieurs des pompes (580), la source d'énergie et le dispositif de commande au couvercle de récipient (304).

9. Système portable de collecte d'urine (300) selon l'une quelconque des revendications 1 à 8, dans lequel les deux pistes opposées (316) sont configurées pour être montées de manière amovible sur le fauteuil roulant sous le siège du fauteuil roulant.

10. Système portable de collecte d'urine selon la revendication 1, dans lequel le couvercle de récipient (304) comprend les deux fentes opposées (308) .

11. Système portable de collecte d'urine selon la revendication 1, dans lequel le support de récipient (310) comprend :
deux supports de guidage comprenant chacun l'une des deux pistes opposées (316) ; et
deux blocs de montage ou plus, chaque support de guidage des deux supports de guidage étant fixé à au moins un bloc de montage des deux blocs de montage ou plus, chaque bloc de montage des deux blocs de montage ou plus comprenant un trou traversant ou un alésage dimensionné pour accueillir une partie du cadre d'un fauteuil roulant.

12. Procédé d'assemblage du système portable de collecte d'urine (300) selon l'une quelconque des revendications 1 à 11, le procédé comprenant :
la fixation de manière amovible du support de récipient à un fauteuil roulant ;
le coulissement des deux pistes opposées dans les deux fentes opposées de l'ensemble de collecte d'urine pour monter l'ensemble de collecte d'urine sur le support de récipient fixé au fauteuil roulant ;
le positionnement d'un dispositif de collecte d'urine à proximité de l'urètre d'un utilisateur ; et
le raccordement fluidique du dispositif de collecte d'urine au récipient de collecte d'urine à l'aide d'un premier conduit.
